(19) Europäisches Patentamt
European Patent Office
Office européen des brevets

(11) **EP 4 084 731 B1**

(12) **EUROPEAN PATENT SPECIFICATION**

(45) Date of publication and mention
of the grant of the patent:
**11.02.2026 Bulletin 2026/07**

(21) Application number: **20839229.0**

(22) Date of filing: **30.12.2020**

(51) International Patent Classification (IPC):
*A61C 1/08* (2006.01)   *A61C 8/00* (2006.01)

(52) Cooperative Patent Classification (CPC):
**A61C 1/084; A61C 8/0089**

(86) International application number:
**PCT/EP2020/025603**

(87) International publication number:
**WO 2021/136590 (08.07.2021 Gazette 2021/27)**

(54) **IMPLANT DRIVER WITH SIMULTANEOUS CONTROL OF DEPTH AND ROTATION OF THE IMPLANT ACCORDING TO A DIGITAL PLANNING**

IMPLANTATTREIBER MIT GLEICHZEITIGER KONTROLLE DER TIEFE UND ACHSDREHUNG DES IMPLANTATS ENTSPRECHEND EINER DIGITALEN PLANUNG

OUTIL DE SERRAGE D'IMPLANT AVEC CONTRÔLE SIMULTANÉ DE LA PROFONDEUR ET DE LA POSITION EN ROTATION DE L'IMPLANT SELON UNE PLANIFICATION NUMÉRIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.12.2019 EP 19220151**
**08.04.2020 EP 20168824**

(43) Date of publication of application:
**09.11.2022 Bulletin 2022/45**

(73) Proprietor: **Dentsply Implants NV**
**3500 Hasselt (BE)**

(72) Inventors:
• POLSPOEL, Wouter
1320 Hamme-Mille (BE)
• CLAES, Ward
3990 Peer (BE)

(74) Representative: **Taor, Simon Edward William**
**Venner Shipley LLP**
**200 Aldersgate**
**London EC1A 4HD (GB)**

(56) References cited:
**WO-A1-2010/125593      US-A1- 2005 147 478**
**US-A1- 2009 011 382      US-A1- 2010 297 574**

## Description

The present invention relates to systems and devices for placing implants in the mouth of a patient and for preparing tools suitable for placing implants in the mouth of a patient.

## Background

The use of implant drivers in combination with a patient-specific surgical template to place dental implants in a predetermined position is a common method in dental implant treatments. Currently, however, there are no commercially available guided surgery instrument kits on the market that allow placing the implant into its predetermined virtually planned position with simultaneous control over the vertical position and the rotation about its longitudinal axis. In most commercialized guided surgery systems, the implant is either placed to its predetermined virtually planned vertical position, or to its predetermined virtually planned rotational position, but not both. The reason for this is that current commercialized instruments for guided surgery do not force the implant to follow a predetermined virtually planned spiral path. The spiral path followed by the implant during implant placement with current commercialized systems is determined by the position of the implant at the moment its thread 'grabs' into the bone, and by the thread design of the implant. Referenced prior art, which is currently not commercialized, does describe implant drivers that would allow for spiral controlled placement of implants leading to repeatable depth and rotation of the implant's final position, however without having the spiral predefined in a virtually planned way.

WO2010/125593 discloses a guided dental implant positioning system, of the kind making use of a surgical guide provided with one or more holes or sleeves in correspondence of the implants insertion points and for controlling the orientation of insertion of the implants, comprising means for simultaneously controlling the position, the final insertion depth, the insertion axis direction and the rotation angle around said axis of each implant, through the setting up of the rotation angle around its own axis of each implant at the beginning of its insertion into the osteotomic seat. Means for simultaneously controlling the depth and axis direction of realisation of osteotomic seats is also disclosed.

EP2153797 an accessory which is made as a positioning jig, wherein said accessory is provided with passages (25) for screws (22) that can be screwed in said implants (7), these passages (25) and said accompanying screws (22) having such a shape that when the screws (22) are tightened they can be forced into a specific position and an accessory in the shape of a drill jig (6) in which one or several ducts (9) are formed for drills, which are provided in the drill jig (6) in function of the computer planning.

WO2007/129955 relates to a device and a method for securing a dental implant in the bone tissue of a patient. The device comprises an internally threaded guide sleeve with an interlock portion that can engage an interlock portion on a tubular mounting guide. A holder comprises a screw complementary to the thread of the guide sleeve. The holder is arranged to secure an implant at one end of the holder. The tubular mounting guide is placed in a hole in a surgical template and cemented in the hole in a desired angular position. The template is placed in the mouth of the patient. Through the hole in the template, a hole into the bone is drilled. The dental implant is secured on the holder and the guide sleeve placed in the hole of the template such that the respective interlock portions (interlock. The holder is screwed through the guide sleeve until the implant is screwed into the bone. The application also relates to a method for making the surgical template.

## Summary of the invention

The present invention pertains to an implant driver assembly as defined in the appended claims 1 to 6.

Embodiments of the present disclosure provide both the design of a surgical template and/or a set of instruments to create and use this template or these instruments to place a dental implant into a position which is a predetermined virtually planned position. This position can be achieved through digitally planning a virtual implant position with respect to digital clinical data of a patient's jaw resulting in the actual definition of the implant position in the jaw.

An aim of embodiments of the present invention is to control simultaneously the position, the final insertion depth, and the rotation angle around the axis of each implant when it is installed in the jaw of a patient.

Another aim of embodiments of the present invention is to be able to place an implant with the implant driver and after reaching the correct vertical position (i.e. correct insertion depth in the jaw) there are markings to show that the implant driver should not be screwed further resulting in damage to the jaw bone. Instead the implant and the driver reach not only a correct vertical position but also simultaneously the correct rotational position whereby further vertical movement is not necessary. This prevents likely damage to the jawbone. Parts of the system cooperate to achieve the correct position of an implant surgically.

The present description also relates to a kit of parts which is not covered by the appended claims, comprising a digital virtually planned implant stored on a signal storage device, an implant driver with one or more first markers, an implant to be placed and a guiding element of a surgical template including one or more second markers, so that alignment of the one or more first markers on the implant driver when it is connected to the implant and one or more second markers on the surgical template define a spiral motion of the implant when it is being inserted in a jaw bone using the implant driver, the implant driver and surgical template also including at least one of the one or more first and at least one of the one or more second markers for indicating the implant driver's final vertical position so that the implant driver's final vertical position is such that the implant, when placed, is in the same vertical and rotational or rotationally symmetric position as defined in the digital virtually planned implant.

By a combined and predetermined position of markers on an implant driver and on a surgical template especially on a guiding element such as a bushing or a sleeve in the bushing, embodiments of the present invention can, when these markers are aligned, define a spiral insertion path of the implant driver that can bring an implant into a position which corresponds to a rotational or rotationally symmetric position. This can be related to a predetermined virtually planned implant position. In addition to the rotationally symmetric position, optionally a vertical position or depth of the implant can also be achieved. The guiding element can have a smooth bore such as a smooth bore bushing or a smooth bore sleeve within the bushing which has a smooth inner surface e.g. without a thread. A sleeve may be fitted within the bushing in which case the sleeve has a smooth bore but the bushing may not require a smooth inner surface in that case.

The bore of a guide element on the surgical template such as a bushing in embodiments of the invention can have one, two three four or up to five pins or screws which enter into the bore and are used as a second marking on a guide element. The pins or screws can be tractable or can be embedded in the guide element such as a bushing. The pins or screws can be thread followers.

Embodiments of the present invention have the advantages:

(i) no need for an internal thread on the inner surface of the guiding element of the surgical guide such as a smooth bore of a bushing or sleeve in the bushing in the surgical guide.

(ii) easier retrieval of the implant driver after the implant has been placed,

(iii) the possibility to use other tools through the bushing without the risk of damaging those tools and

(iv) direct integration into the surgical plan and potential programming of the dental handpiece.

In one aspect of the present invention, an implant driver is provided with a set of implants or with an implant to be placed in a patient's jaw, the outer surface of the implant driver having a rotational marker. Optionally there can be a depth marker for the depth of insertion of an implant in the jaw of a patient. The rotational marker can be distributed over the surface of the implant driver, e.g. in the form of a spiral. Use of a distributed rotational marker has the advantage that at least a part of it can be viewed by the dentist or dental surgeon at any time. The distributed marker can include a variety of marker elements such as colored bands, lines, piece-wise linear markers such as lines of dots or dots and dashes. The rotational marker can be placed in or on the implant driver by any suitable process such as printing, painting, milling, laser ablation, engraving, etching. For example, the rotational marker can be indented, scratched, milled into the surface of the implant driver, e.g. it can be a thread. The position of the markers on the implant driver with respect to markings on the guide element such as on a bushing with optionally a sleeve, is determined by the design specification of the implant driver, the design specification of the surgical template and the position of the virtually planned implant. The latter is patient-specific and, thus, determines the patient - specific character of the markers' position.

An implant depth marker can be provided by a localized marker element, for example any suitable design such as one or more lines, piece-wise linear markers such as lines of dots, one or more localized engravings, one or more localized etchings, one or more localized printings or paintings, one or more localized millings, or similar. The markers on the implant driver preferably align with at least one marker on the surgical template e.g. on the guiding element such as the bushing when the implant is in the final and correct rotation and depth position. First markers on the implant driver and second markers on the guide element of the template define a spiral motion. The first and second markings can interact with each other, co-operate with each other or coordinate with each other to define the rotational start and end point and for example all points between.

A vertical stopping position does not need necessarily that there is co-operation between a first marker on the implant driver and a second marker on the guide element such as a bushing. For instance, a further embodiment of the present invention provides an implant driver that has a thread that stops at a certain position along its length. This can be used to determine the final vertical position of the implant without a need for a second marker on the surgical template.

The marker on the bushing of the surgical template includes one or more retractable or embedded thread followers at a patient - specific location. The thread follower co-operates with the rotational marker on the implant driver that is e.g. also a thread or spiral groove. The thread can be formed in or on the surface of the implant driver. The position of the thread follower with respect to the guide element such as a bushing is determined by the design specification of the implant driver, the design specification of the guide element such as the bushing and the position of the virtually planned implant. The latter is patient-specific and, thus, determines the patient - specific character of the thread follower's position. The thread follower is a marker that can facilitate or even enforce the alignment with the rotational marker on the implant driver, e.g. the implant driver thread. As the thread follower is inserted into the thread on the implant driver or the thread on the implant driver is inserted into the thread follower on the implant driver, the movement of the implant driver with respect to the surgical template is controlled by the position of the thread follower and the sliding movement of the thread follower where it is inserted in or on the thread of the implant driver.

In some embodiments of the present invention the implant driver's thread can correspond with the thread of the implant i.e. have the same pitch of the thread. Furthermore, the implant driver can also comprise a marker for indicating an implant driver's correct final position, e.g. rotationally and/or vertically. When the implant is placed with the implant driver in this particular final position, the implant has reached the same vertical and rotational or rotationally symmetric position as was defined for the virtually planned implant. For this reason this position is called the final position. Note that an implant connection has an x-fold rotational symmetry, e.g. a 6-fold symmetry when the implant connection has a hexagonal shape. It can be allowed that the final implant rotational position around its vertical axis is a multiple of the virtually planned rotational position. For example, for a hexagonal connection, the final implant can be rotated around its longitudinal axis by an angle such as 60°, 120°, ... with respect to the virtually planned rotational orientation around its longitudinal axis. The implant driver and bushing design can vary to reach a similar or an identical result. For example, one could have the thread follower on the implant driver and the thread on the bushing.

The present document describes a method for designing, and/or manufacturing and/or using a surgical template with patient specific markers and/or an implant driver with markers, such that an implant can be placed according to a predetermined virtually planned position.

In another aspect of the present invention specific mathematical calculations are presented as one way to determine the marker positions. Other methods of deciding on the marker positions which do not use the mathematical calculation are described herein.

Also described is a method to determine the number of implant driver turns or rotations required to reach a final position of the implant matching a virtually planned implant position. This number can then be programmed into a motorized handpiece used for driving in the implant driver, such that reaching the final position does not require anymore the use of markers indicating the final implant driver position.

Embodiments of the present invention may solve one or two main problems that exist in the referenced prior art. For example, the implant driver and the surgical template in accordance with embodiments invention do not require two threaded interacting components. The first problem is related to the fact that known methods require threading of both the bushings of the surgical template (or an additional threaded sleeve mounted to the surgical template, e.g. within the bushing) and the implant drivers. Such a combination of two threaded interacting components has several disadvantages. Firstly, it does not allow for easy decoupling of the implant driver from the implant when the final position of the implant is reached. Indeed, the implant driver can typically only be decoupled from the implant by translating it along its longitudinal axis away from the implant. Such motion is impossible when the threaded implant driver is also coupled to the thread of the surgical template's bushing. An aspect of some embodiments of the present invention include one or more thread follower(s) that is (are/can be) retractable. The thread follower can be a simple pin. Such a pin has a local penetration in, or a point contact in the threaded groove on the implant driver. Retraction allows for translational motion of the implant driver necessary for decoupling it from the implant. Secondly, a threaded bushing or a threaded additional sleeve in the surgical template is not optimal for guidance of other, typically faster rotating, surgical instruments such as drills. For guidance of these instruments, a smooth inner surface of the bushing (i.e. smooth bored bushing) or the smooth bore of a sleeve when a sleeve within the bushing is preferred. In short, the introduction of one or more discrete markers (such as a guiding pin acting as a thread follower) in the bushing of the surgical template, instead of a fully threaded bushing is deemed a superior concept in comparison with referenced prior art.

The bore of a guide element on the surgical template such as a bushing in embodiments of the invention can have one, two three four or up to five pins or screws which enter into the bore and are used as a second marking on a guide element. The pins or screws can be tractable or can be embedded in the guide element such as a bushing. The pins or screws can be thread followers.

A second problem that exists with the referenced prior art is that it does not include a method to determine the marker positions based on a virtual implant planning. As a result, prior art disclosures do not provide means for placing an implant that matches, both in vertical depth and rotational or rotational symmetrical position, a virtually planned implant position. Embodiments of the present invention allow for the placement of an implant at a position and with an orientation matching a virtually planned position rotation or rotational symmetry. A matching of a virtually planned position can be both in depth and in rotational position. This is in contrast to the referenced prior art, whereas embodiments of the present invention allow for more accurate placement of virtually planned and virtually designed patient - specific prosthetic components, such as abutments and crowns, in the same treatment session used for placing the implant(s). Since both the vertical (or depth) and rotational position of the implant to be placed are controlled, the position of the placed patient - specific prosthetic components will match more accurately their virtually planned position. In referenced prior art, the implant position is predetermined and repeatable only after the surgical template is manufactured. Thus, the referenced prior art only allows for preparing the prosthetic components in a manual manner (e.g. by implant (analog) placement in a physical model using the same surgical template) but not in a digital manner. For this reason, the method for determining the patient - specific position of the discrete marker(s) in the surgical template guiding element such as a smooth bored bushing, based on a virtually implant position and based on predetermined markers on the implant driver, is deemed a superior concept.

Described herein is also a kit of parts comprising a digital virtually planned implant stored on a signal storage device, an implant driver with one or more first markers, an implant to be placed and a guiding element of a surgical template including one or more second markers, so that alignment of one or more first markers on the implant driver (which is connected to the implant when it is to be driven in) and one or more second markers on the surgical template defines a spiral motion of the implant when it is being inserted in a jaw bone, the implant driver and surgical template also including at least one of the one or more first and at least one of the one or more second markers for indicating the implant driver's final vertical position so that the implant driver's final vertical position is such that the implant when placed is in the same vertical and rotational or rotationally symmetric position as the digital virtually planned implant. The above is preferred to only a marker of final position as this is not sufficient because this definition would include the situation where one can place the implant with the implant driver in a normal way and then after reaching the correct vertical position first, screwing the implant driver further into correct rotational position while blocking further vertical movement. This would most likely damage the bone.

The guiding element can have a bore, the bore being smooth. This provides the advantage that it is easier to remove the implant driver from the implant after it has been placed in the jaw of a patient. The bore of a guide element on the surgical template such as a bushing in embodiments of the invention can have one, two three four or up to five pins or screws which enter into the bore and are used as a second marking on a guide element. The pins or screws can be tractable or can be embedded in the guide element such as a bushing. The pins or screws can be thread followers.

The one or more first markers can be one or more distributed marker. This makes it more easily viewable by a dental surgeon or dentist.

The one or more first markers can comprise a thread in or on an outer surface of the implant driver. The thread can have a thread start and a thread entrance. A thread is easy to manufacture.

The one or more second markers can comprise a thread follower for following the thread which is in or on the outer surface of the implant driver. A thread follower provides positional stability.

The thread follower can be one or more pins, some or each of which is or can be removable from the guiding element of the surgical template.

The guiding element can be a smooth bored bushing. This makes removal of the implant driver from the implant easier after the implant has been inserted in the jawbone.

The implant driver can narrow at one end so that the thread follower disengages from the thread. This makes removal easier of the implant driver from the implant after the implant has been inserted in the jawbone.

## Brief description of the drawings

Figure 1A shows schematically a combination of an implant driver, a virtually planned implant, a surgical template with a guiding element in the form of a smooth bored bushing and a thread follower, according to an embodiment of the present invention.

Figure 1B shows a detail of Figure 1A.

Figure 2A shows a combination of an implant driver and placed implant in final position and a guiding element in the form of a smooth bored bushing of a surgical template, according to an embodiment of the present invention.

Figure 2B shows a detail of Figure 2A.

Figure 3 shows a part of an implant driver with a thread, thread entrance and thread start, a part of an implant, and a guiding element in the form of a smooth bored bushing with a thread follower in the form of a guiding pin according to an embodiment of the present invention.

Figure 4 shows an implant driver with a thread, thread entrance and thread start, an implant, and a guiding element with a thread follower in the form of a guiding pin according to an embodiment of the present invention.

Figure 5 shows an implant driver with a thread, thread entrance and thread start, a part of an implant, and a guiding element with different thread followers in the form of guiding pins, according to an embodiment of the present invention.

Figure 6 shows an implant driver narrowed near the top with a thread, thread entrance and thread start, a part of an implant, and a guiding element with an embedded thread follower, according to an embodiment of the present invention.

Figure 7 shows a part of an implant driver that is in virtual final position, i.e. connected to the virtually planned implant, according to an embodiment of the present invention.

Figure 8 shows a part of an implant driver with spiral defining markers such as a spiral line and a set of dots spaced by the pitch of the spiral and a final position marker, a guiding element with a marker to be aligned with the spiral marker on the implant driver and a final position marker to be aligned with the final position marker of the implant driver, according to an embodiment of the present invention.

**Definitions**

**"Spiral insertion path":** when a tooth implant is inserted into a jaw bone an implant driver is rotated which screws the implant into the jaw bone. A point on the implant driver will therefore rotate and move linearly along a direction parallel to the axis of rotation whereby the combined movements generate a spiral path for the point.

"Smooth bored" means all embodiments of the present invention not having a thread or other surface irregularities on the inside of a guide element on a surgical template such as a bushing. The bore of a guide element on the surgical template such as a bushing in embodiments of the invention can have one, two three four or up to five pins or screws which enter into the bore and are used as a second marking on a guide element. The pins or screws can be tractable or can be embedded in the guide element such as a bushing. The pins or screws can be thread followers.

**"Distributed marker"** means a marker that is spread over the surface e.g. of the implant driver so that a part of the marker can be seen by a dentist or dental surgeon independent of the rotational position of the implant driver. Such a marker can be present at any position around the surface e.g. of the implant driver, e.g. a thread traverses 360° of the surface e.g. of the implant driver.

**"Localized marker"** means a marker whose are is small so that the marker is at a particular location rather than being spread over a surface. A typical localized marker would be a point or a line.

**Description of illustrative embodiments**

Herein described is the use of virtual planning software for planning the *in silico* position of an implant, i.e. to make a preoperative plan. An input to the preoperative plan is preferably one or more images obtained from one or more volumetric scans such as an MRI scan, a conventional or cone beam (CB) computer tomography (CT) scan of the patient's anatomy, e.g. the jawbone and dentition. Optionally other types of scans such as an optical scan of an *in vitro* model or an intraoral optical scan of a patient's mouth can be used alone or in combination with other scans. From the one or more scans, a digitization of the patient's anatomy, e.g. dentition, is obtained, e.g. by using software and conventional techniques known to the skilled person. In the preoperative plan, the digitizations of the patient's anatomy, e.g. bone and dentition, are combined and the dental surgeon or practitioner can plan virtually the implant positions and orientations in their optimal or best possible clinical and esthetical position.

Herein described is the generation and use of a digital preoperative plan, the design of a surgical template or a set of instruments as well as a method to create and use this surgical template or these instruments to place a dental implant into a predetermined virtually planned digitally defined position.

The predetermined virtually planned digitally defined position of an implant may be stored digitally in a random access memory or on a signal storage medium such as an optical disk, a magnetic tape, a hard drive, a semiconductor solid state memory such as a flash drive, etc.

By having predetermined positions of markers for example on both of an implant driver and on a surgical template, or on one of these, embodiments of the present invention can define a spiral insertion path of the implant driver that can bring an implant into a position which corresponds in rotational or rotationally symmetric position to a pre-determined virtually planned implant position. Optionally, the position can correspond in both vertical and rotational or rotationally symmetric position to a pre-determined virtually planned implant position. The latter position is called the final position and may be indicated by an additional (set of) marker(s) on either, each or both of the implant driver and a guiding element that are aligned upon reaching the final position.

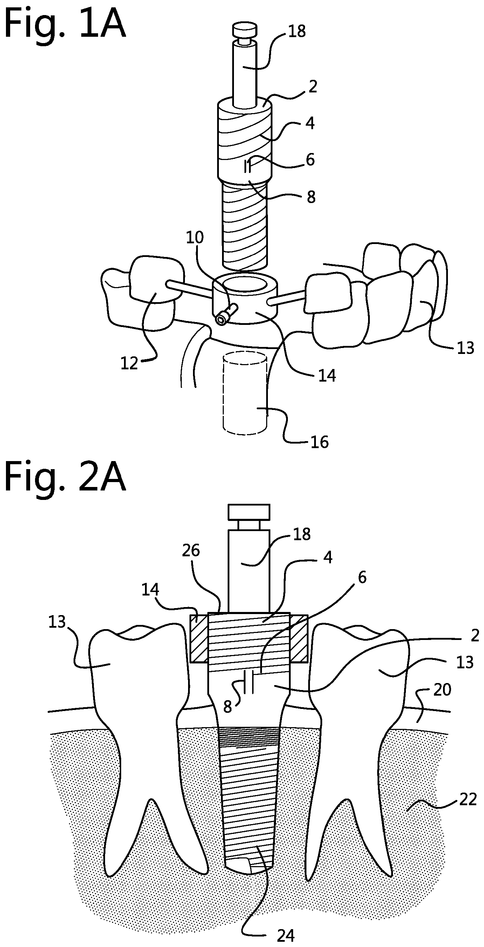

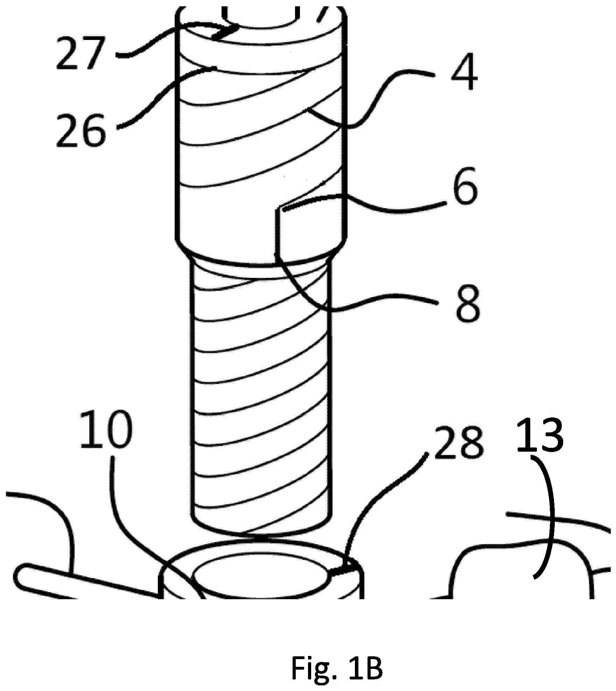

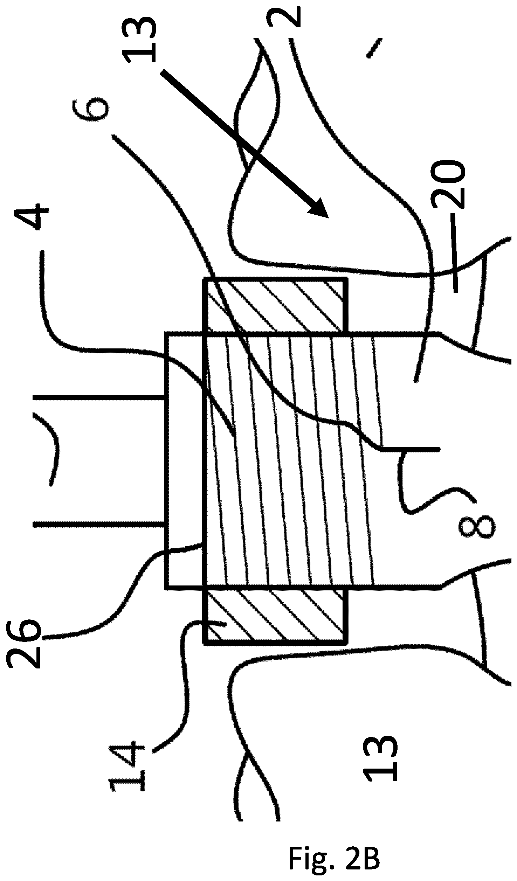

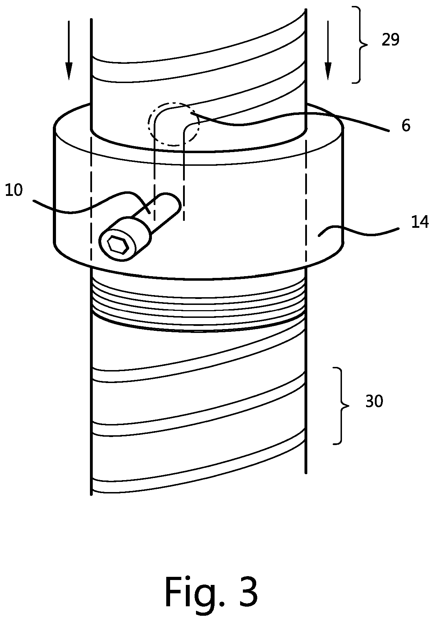

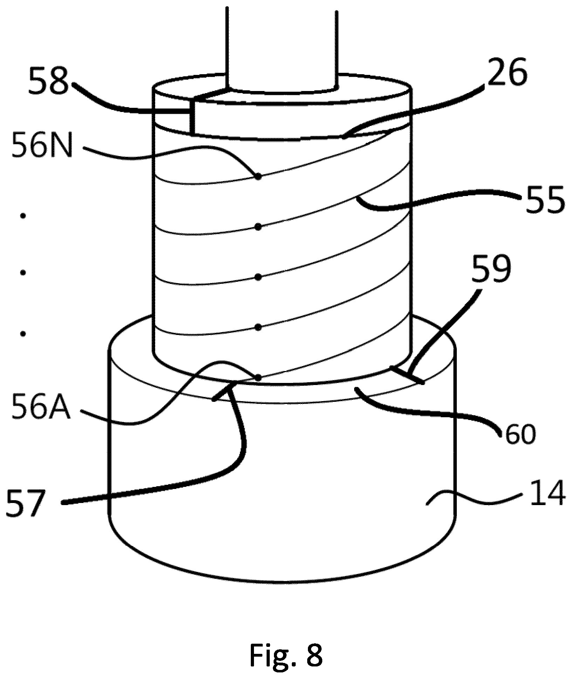

Figures 1A and 1B show an implant driver 2 with a shank 18 and an implant driver's connection shown schematically to a virtual implant position 16 in accordance with an embodiment of the present invention. Figures 2A and 2B show an implant driver 2 with a shank 18 and an implant driver's connection to an actual implant 24 in accordance with this embodiment of the present invention. When an implant driver 2 is used to install an implant 24 in accordance with this embodiment it is done using a first marking such as a thread 4 for example as at least one distributed rotational marker which allows the positioning of an implant 24 into the jaw when using the thread 4 to determine the final position. Thus, as the driver 2 is rotated the implant 24 enters further into the jaw. Any point on the implant driver 2 will follow a spiral path as the driver 2 is rotated and moves downwards towards the jaw or into the jaw. The correctly terminated spiral path of the implant driver's connection to achieve an implant position that coincides in rotation and optionally both in depth and rotation to a predetermined virtual implant position, can be indicated by aligning a combination of markers on both the implant driver (e.g. first marking such as a thread 4 (see Figure 1A), or a drawn line 26 (see Figure 1B) or 55 (see Figure 8)) or a set of dots in a line 56A to N (see Figure 8), a thread start 6 or a thread entrance 8 (see Figure 1B)...) and localized markers (e.g. second marking such as thread follower 10, or a drawn line 27 or 28 (see Figure 1B) or 57, 58, 59 (see Figure 8, ...) that have a patient - specific position on a guiding element (e.g. on a smooth bored bushing 14 (see Figure 1A)) of a surgical template 12. The first and second markings can be aligned with each other, or can co-operate with each other, interact with each other, be coordinated with each other, interoperate with each other to bring the implant 24 into the correct final position. Thereby, the markers, such as a thread 4 (see Figure 1A), or a drawn line 26 (see Figure 1B), a spiral line 55 (see Figure 8) or a set of dots in a line 56 (see Figure 8) or a thread start 6 or a thread entrance 8 etc. on the implant driver 2 define the pitch 29 of a spiral path of the implant driver 2 (see Figure 3). The spiral path's pitch 29 (see Figure 3) can be optionally identical to the pitch 30 (see Figure 3) of the implant 's lead screw thread to avoid damaging of the implant 2 or the jawbone upon placing the implant 24. The alignment of these distributed spiral defining markers on an implant driver 2 and localised markers on a surgical template 12 such as on a smooth bored bushing optionally with an inserted sleeve define the spiral movement of the implant driver 2 and its final position, and, thus, the implant 24 connected to it. This places the implant 24 according to a predetermined and virtually planned digitally defined implant position, including its predetermined virtually planned final rotation position around its longitudinal axis and/or its depth within the jaw.

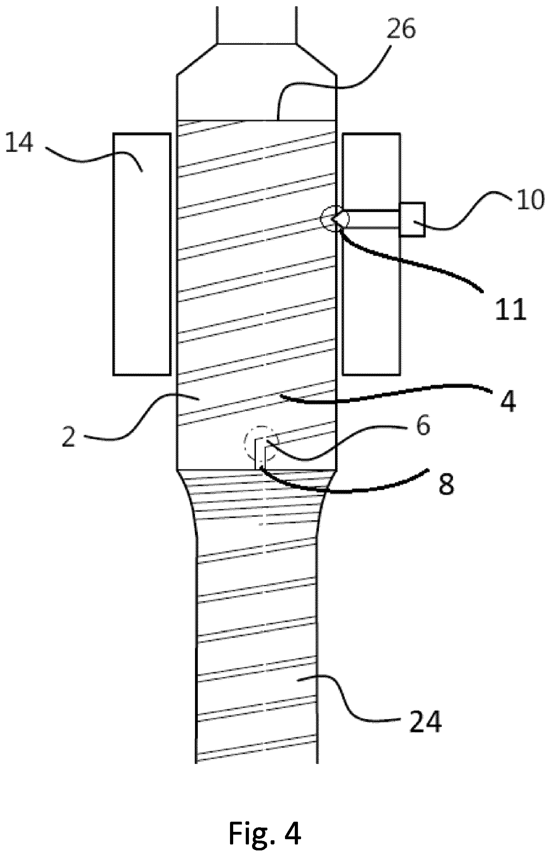

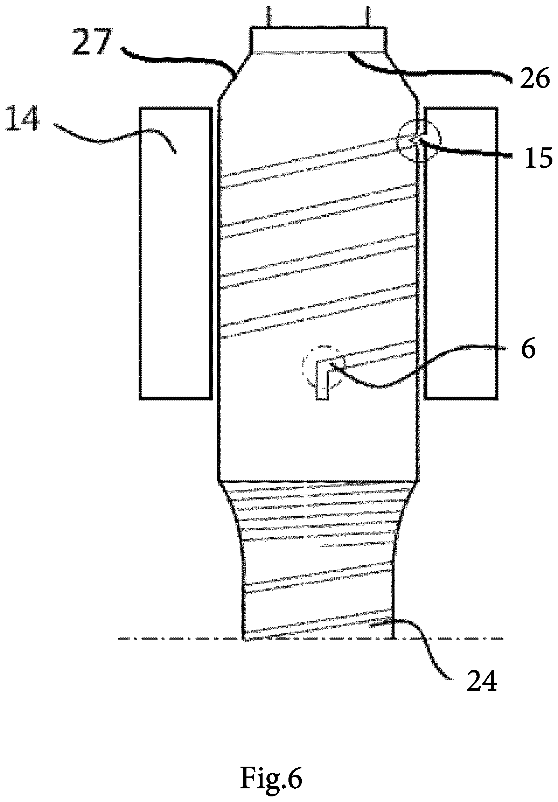

A surgical template 12 fits onto the oral anatomy of a patient such as teeth 13. Methods to achieve this are known to the skilled person. The surgical template 12 has a guiding element (e.g. a smooth bored bushing 14). The guiding element such as the smooth bored bushing 14, will locate over the position of a missing tooth of the patient and, hence, at a virtual implant position 16 where an implant 24 is to be placed. In an embodiment of the invention, the spiral defining first marker on the implant driver 2 is a thread 4, with a thread start 6 and a thread entrance 8 (seen best in Figure 1B). The function of the thread entrance 8 and the thread start 6 is to facilitate the alignment of such a marker on the implant driver 2, e.g. the thread 4, with a localized second marker on the guiding element (such as on a preferably smooth bored bushing 14). This can be a thread follower defined by the apex 11 of a guiding pin/screw 10 or 15 (see Figure 4 or Figure 6) that is assembled to a smooth bored bushing 14 by means of a (e.g. threaded in case of a screw) hole in the smooth bored bushing 14. The position of the pin/-screw's apex 11 is patient - specific and depends on the position of the virtually planned implant 16, the design specification of the implant driver 2 including its markers, 4, 6, 8, 26, 27, 28, 55, 56, 57, 58, 59 and the design specification (e.g. vertical position) of the guiding element (such as smooth bored bushing 14) of the surgical template 12. The depth with which the thread follower 10 penetrates into the guiding element such as the smooth bored bushing 14 is preferably adjustable so that the apex 11 of the thread follower 10 enters and glides in the thread 4 while still allowing the implant driver 2 to be rotated and to drive the implant 24 into the jaw bone 22 of the patient through the gingiva 20 (see Figure 2A). When the first and second markings on both the implant driver 2 and the smooth bored bushing 14 are aligned, e.g. when the apex 11 of the thread follower follows the thread 4, the spiral path of the implant driver is such that it will drive the implant 24 into the bone in such a way that the implant 24 can be placed according to a virtually planned position, at a rotational position around its longitudinal axis or optionally both in depth and rotational position. This is called the final position.

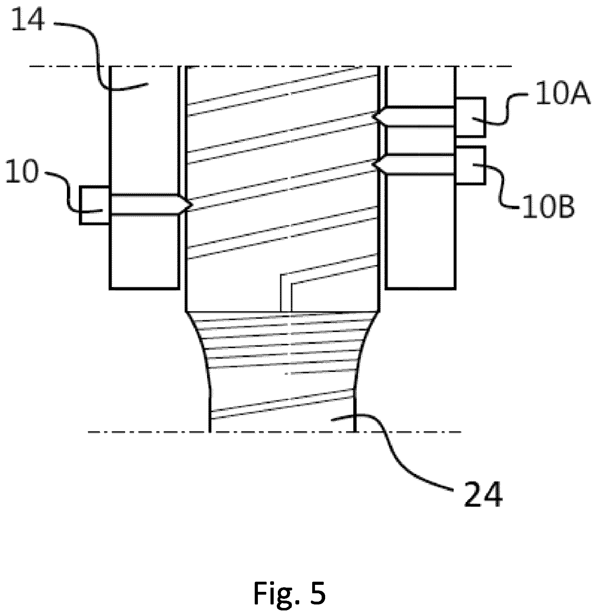

More than one apex 11 or guiding pin/screw 10 can be used as shown in Figure 5 - 10A, 10B, 10. However, in order for these guiding pin/screws 10A and B to avoid blocking the rotation at the start they can be made retractable such that they are generally in a withdrawn position and are only moved into position engaging with the groove 4 after the guiding pin/screw 10 has entered into the spiral groove 4.

The bore of a guide element on the surgical template such as a bushing in embodiments of the invention can have one, two three four or up to five pins or screws which enter into the bore and are used as a second marking on a guide element. The pins or screws can be tractable or can be embedded in the guide element such as a bushing. The pins or screws can be thread followers.

The final implant position is achieved, for example as indicated by aligning a final position marker 4, 6, 8, 26 and/or a rotational marker 27 on the implant driver 2 with final position markers 28, (top surface) 60 on the surgical template 12. For example, a localized vertical position indicating line 26 on the implant driver 2 (see Figure 1B) can be aligned with the top 60 of the smooth bored bushing 14, or a localized rotational final position indicating line 27, 58 (see Figure 8) on the implant driver 2 can be aligned with the localized rotational final position indicating line 28, 59 (see Figure 8) on the smooth bored bushing 14 of the surgical template 12. Final position markers on the implant driver 2 and surgical template 12 can exist in many forms such as lines, dots, ridges, valleys, colored bands, physical stops, physical shape changes, etc. that can be created in different ways. They can be drawn, engraved, milled, painted, printed etched, laser ablated or added onto marker elements in any other way, etc.

An alternative embodiment includes an implant driver 2 that is narrowed near the top such that the thread follower 10 loses contact with the implant driver 2 before the final position is reached. This final position can be indicated by a final position marker 26. When this happens, the spiral movement is no longer dictated by the cooperation of the implant driver 2 and thread 4 with the thread follower 10, but by the (thread of the) implant 24 with the bone. This latter embodiment allows to easily detach the implant driver 2 from the implant 24 after the final position is reached, e.g. in cases where an embedded (i.e. non-retractable) thread follower 10 is used.

Alternative embodiments of the present invention can include an implant driver 2 where the thread 4 can be replaced by a different kind of distributed marker that also defines a spiral, the spiral path's pitch 29 being optionally identical to the implant's lead screw thread pitch 30 (see Figure 3) to avoid damaging of the implant 24 or jaw bone upon placing the implant 24. These implant driver markers 4, 6, 8, 26, 27, 55, 56 can take many forms such as spirals, dots, lines which can be drawn, engraved, etched, painted, printed, laser ablated, milled, or added onto elements or by any other means, ... Examples are a physical screw thread 4 (e.g. an engraved spiral), a laser marked spiral, a line of dots 56A... 56N (see Figure 8) spaced vertically and all defining a pitch 29, etc. Preferably the thread marker 4 co-operates with a thread follower 10 which is located on the surgical template 20 and passing through the guide such as the smooth bored bushing 14. The patient-specific markers 28, 57, 59, 60 on the guiding element of the surgical template, such as the smooth bored bushing 14, can also take many forms, such as any of line(s), dot(s), groove(s), hole(s), etc. that is drawn, engraved, painted, printed, laser ablated, milled, added by any other means, etc. on the guiding element such as the smooth bored bushing 14 of the surgical template 12. Such type of markers 28, 57, 59, 60, when aligned with the markers 4, 6, 8, 26, 27, 55, 56 on the implant driver 2, allow for visual enforcement and feedback of the position of the implant driver 2 and, thus, the implant 24 connected to it, along an insertion path that places the implant 24 exactly according to a predetermined virtually planned implant position. The final position can be indicated by alignment of final position markers 4, 6, 8, 26, 27 on both the implant driver 2, such as a line 27, 58, and the guiding element, such as a line 28, 60.

**Description of a method** Presented herein is a method for designing, and/or manufacturing and/or utilizing a surgical template 12 and a smooth bored guide 14 with patient specific localized markers 28, 57, 59, or 60 and/or an implant driver 2 with markers 4, 6, 8, 26, 27, some of which can be distributed markers. Distributed markers can be present around all of the circumference of the implant driver. The markers are used such that an implant 24 can be placed according to a predetermined virtually planned position. This predetermined virtually planned position can be achieved through digitally planning an implant position with respect to digital clinical data which can be obtained by any suitable scanning techniques such CAT-scan, X-ray, MRI, CBCT, optical or intra-oral scan, or any combination thereof.

A method for determining the allowed positions of the surgical template guiding element's markings, such as the apex 11 of a pin - shaped thread follower 10, is provided with the following steps.

- Step 1: Manufacture an implant driver 2 that has one, some or all of markers 4, 6, 8, 26, 27, 55, 56, 58 in the design specification of the implant driver 2. One or more of these markers can be distributed markers, e.g. that define a spiral including following specifications:

  ○ The longitudinal axis of the spiral coincides with the longitudinal axis of the implant driver 2.
  ○ The pitch 27 of the spiral optionally coincides with the pitch 30 of the thread of the implant(s) with which the implant driver 2 is compatible.
  ○ Optionally, the markers 4, 6, 8, 26, 27, 55, 56, 58 on the implant driver 2 facilitate alignment with the marker(s) 28, 57, 59, 60 on the surgical template 12 e.g. on smooth bored bushing 14. For example, the thread entrance 8 and thread start 6 facilitate alignment with the thread follower 10 on the surgical template 12, e.g. on bushing 14 to establish a predefined initial position of the thread start 6 and, thus, the implant driver / implant assembly.

- Step 2 (before or after step 1): Virtually plan an implant position for a patient's jaw with respect to digital clinical data obtained from the patient.
- Step 3: Based on the virtual implant position and the design specification of the implant driver 2, define the virtual spiral defined by the position of the implant driver's markers 4, 6, 8, 26, 27, 55, 56 on the implant driver 2 in its virtual final position, i.e. connected to the virtually planned implant.
- Step 4: Define the position of the marker(s) 28, 57, 59, 60 in the design of the surgical template's guiding element (e.g. smooth bored bushing 14) such that it can be aligned with the spiral path as defined in step 3.
- Step 5: Manufacture the surgical template 12 including the marker(s).
- Step 6: Install the surgical template 12 on the patient's anatomy, create the osteotomy, connect the implant driver 2 to the implant, insert the implant driver / implant combination in the guiding element 14 of the surgical template 12, align the markers 28, 57, 59, 60 on the guiding element 14 of the surgical template 12 with the markers 4, 6, 8, 26, 27, 55, 56 of the implant driver 2, drive the implant 24 into the bone while keeping the markers 4, 6, 8, 26, 27, 28 on both the implant driver and guiding element aligned. Stop the placement of the implant 24 when the final position is reached, indicated by final position markers on both the implant driver and the guiding element 4, 6, 8, 26, 27, 28.

Presented herein is a method for programming, in a dental hand piece setup, the rotation required to place an implant into a digitally predetermined position:

- Step 1: Manufacture an implant driver 2 that includes one, some or all of markers 4, 6, 8, 26, 27, 55, 56 in the design specification of the implant driver 2 that define a spiral including following specifications:

    ○ The longitudinal axis of the spiral coincides with the longitudinal axis of the implant driver 2.
    ○ The pitch 29 of the spiral optionally coincides with the pitch 30 of the thread of the implant(s) with which the implant driver is compatible.
    ○ the markers 4, 6, 8, 26, 27, 57, 56, 58 on the implant driver 2 facilitate alignment with the marker(s) 28, 57, 59, 60 on the surgical template 12, such as a thread entrance 8 and thread start 6, to allow establishing a predefined initial position of the thread start 6 and, thus, the implant driver / implant assembly.

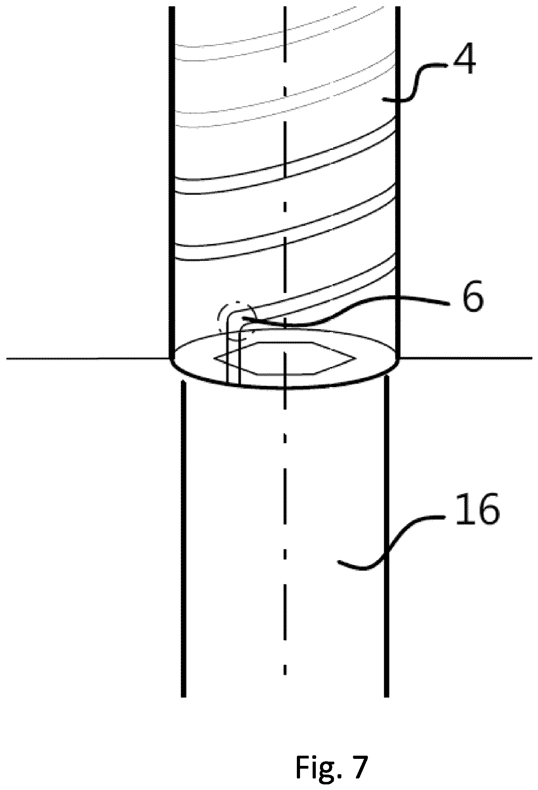

- Step 2: (before or after step 1) Virtually plan an implant position for a patient's jaw with respect to digital clinical data obtained from the patient.
- Step 3: Based on the virtual implant position and the design specification of the implant driver, define the virtual spiral defined by the position the implant driver's markers 4, 6, 8, 26, 27, 55, 56 of the implant driver 2 in its virtual final position, i.e. connected to the virtually planned implant 24. Furthermore, identify the position of a thread start 6 of the implant driver 2 that is virtually connected to the virtually planned implant position. This is the thread start's final position (see Figure 7).
- Step 4: Choose the position of the thread follower/-guiding pin 10 in the design of the surgical template 12, e.g. the position of guiding pin's apex 11, as a point located on the spiral path as defined in Step 3. By definition, upon implant placement, the thread start's initial position will coincide with the position of the thread follower 10. See section "Operating principle" below for more details.
- Step 5: Calculate the polar rotation required to move the thread start's initial position to the thread start's final position (determined in step 3) following the spiral defined by the markers 4, 6, 8, 26, 27, 55, 56 of the implant driver 2. This can be done using the following formula where $\alpha$ is the polar rotation, h is the vertical height (measured along the longitudinal axis of the virtually planned implant) between thread start's initial position and the thread start's final position:

$$\alpha = 2\pi \cdot \frac{h}{pitch}$$

- Step 6: Program this polar rotation into a dental hand piece system such that this rotation can applied to the implant driver 2 after the thread start 6 was moved into its initial position, i.e. thread start 6 coincides with the position of the thread follower 10, e.g. a guide pin's apex position 11.

**Operating principle**

For the sake of clarity, an embodiment of a surgical template 12 with a guiding element in the form of a bushing 14 (preferably smooth bored) with a single guiding thread follower such as a pin 10 is disclosed.

- First a surgical template 12 is designed and manufactured such that a predetermined set of surgical instruments can be guided by a guiding element such as a bushing 14 (preferably smooth bored) that is part of the template 12. This surgical template 12 is used to create an osteotomy that is positioned according to a preoperatively planned virtual position of an implant 24. That virtual implant position is planned with respect to digital clinical data obtained from the patient.
- The guide element or bushing 14 (preferably smooth bored) of the surgical template 12 is designed and manufactured such that it includes a retractable or embedded thread follower 10 such as the apex 11 of retractable pin/screw 10 or embedded pin 15 (see Figure 6), such that the pin / screw's apex 11 will be positioned on the spiral defined by the method.
- After the osteotomy has been created, the implant driver 2 is or can be connected to the implant 24, this assembly being suitable for being moved into, or is then moved into the guiding element such as a bushing 14 (preferably smooth bored) of the surgical template 12. The driver thread entrance 8 is aligned with the apex 11 of the pin/screw 10, 15, the driver 2 is then moved down into the bushing 14 (preferably smooth bored) until the thread start 6 coincides with the pin / screw's apex 11. This is the thread start's initial position.
- Then, the implant driver / implant assembly is moved down in a spiral motion that is forced by the implant driver's thread 4 and the bushing's thread follower 10 since the pin's/screw's apex 11 is following the thread 4 of the implant driver 2. This movement is continued until the thread start 6 and thus the implant diver / implant assembly, is moved into its final position. This final position can be further indicated by aligning final position markers 4, 6, 8, 26, 27, 58 on the implant driver 2, with final position markers on the surgical template, such as line 28, the top 60 of the surgical template 12 and a line 59 on the bushing 14 (preferably smooth bored), respectively.

## Claims

1. An implant driver assembly for placing an implant according to a digital virtually planned implant and implant position in the jaw of a patient, wherein said implant driver assembly comprises at least an implant driver (2) and at least a surgical template, (12) wherein the implant driver having one or more first markers, and being adapted to be aligned with the surgical template, the surgical template having a guiding element (14) and having one or more second markers, so that alignment of one or more first markers on the implant driver when connected to the implant and one or more second markers on the surgical template defines a spiral motion of the implant when it is being inserted in a jaw bone, the implant driver and surgical template also including at least one of the one or more first and at least one of the one or more second markers for indicating the implant driver's rotational or rotationally symmetric position as defined by the digital virtually planned implant position, whereby the guiding element is a bushing (14) with a smooth bore; wherein the one or more first markers comprises a thread (4) in or on an outer surface of the implant driver; wherein the one or more second markers comprises a thread follower (10) for following the thread which is in or on the outer surface of the implant driver; **characterised in that** the thread follower (10) comprises one or more pins that is/are removable or retractable from the guiding element of the surgical template.

2. The implant driver assembly according to claim 1, wherein at least one of the one or more first and at least one of the one or more second markers are for indicating the implant driver's final vertical position.

3. The implant driver assembly according to claim 1 or 2, wherein the digital virtually planned implant and/or implant position in the jaw of a patient are stored on a signal storage device.

4. The implant driver assembly according to any the previous claims, wherein one of the one or more first markers is a distributed marker.

5. The implant driver assembly according to any the previous claims, wherein the thread has a thread start (6) and a thread entrance (8).

6. The implant driver according to any of the previous claims, wherein the implant driver is adapted to pass through the smooth bored bushing. (14).

## Patentansprüche

1. Implantattreiberanordnung zum Platzieren eines Implantats gemäß einer digitalen, virtuell geplanten Implantier- und Implantatposition in dem Kiefer eines Patienten, wobei die Implantattreiberanordnung zumindest einen Implantattreiber (2) und zumindest eine chirurgische Schablone (12) umfasst, wobei der Implantattreiber einen oder mehrere erste Marker aufweist und angepasst ist, um mit der chirurgischen Schablone ausgerichtet zu werden, wobei die chirurgische Schablone ein Führungselement (14) aufweist und einen oder mehrere zweite Marker aufweist, so dass die Ausrichtung eines oder mehrerer erster Marker auf dem Implantattreiber, wenn er mit dem Implantat verbunden ist, und eines oder mehrerer zweiter Marker auf der chirurgischen Schablone eine Spiralbewegung des Implantats definiert, wenn es in einen Kieferknochen eingesetzt wird, wobei der Implantattreiber und die chirurgische Schablone auch zumindest einen des einen oder der mehreren ersten und zumindest einen des einen oder der mehreren zweiten Marker zum Anzeigen der rotations- oder rotationssymmetrischen Position des Implantattreibers einschließen, wie sie durch die digitale, virtuell geplante Implantatposition definiert ist, wodurch das Führungselement eine Buchse (14) mit einer glatten Bohrung ist; wobei der eine oder die mehreren ersten Marker ein Gewinde (4) in oder auf einer Außenfläche des Implantattreibers umfassen; wobei der eine oder die mehreren zweiten Marker einen Mitnehmer (10) zum Folgen des Gewindes umfassen, welches sich in oder auf der Außenfläche des Implantattreibers befindet; **dadurch gekennzeichnet, dass** der Mitnehmer (10) einen oder mehrere Stifte umfasst, der/die von dem Führungselement der chirurgischen Schablone entfernbar oder zurückziehbar sind.

2. Implantattreiberanordnung gemäß Anspruch 1, wobei zumindest einer von dem einen oder den mehreren ersten und zumindest einer von dem einen oder den mehreren zweiten Markern zum Anzeigen der endgültigen vertikalen Position des Implantattreibers dient.

3. Implantattreiberanordnung gemäß Anspruch 1 oder 2, wobei die digitale, virtuell geplante Implantier- und/oder Implantatposition in dem Kiefer eines Patienten auf einer Signalspeichervorrichtung gespeichert ist.

4. Implantattreiberanordnung gemäß einem der vorhergehenden Ansprüche, wobei einer von dem einen oder den mehreren ersten Markern ein verteilter Marker ist.

5. Implantattreiberanordnung gemäß einem der vorhergehenden Ansprüche, wobei das Gewinde einen Gewindeanfang (6) und einen Gewindeeingang (8) aufweist.

**6.** Implantattreiber gemäß einem der vorhergehenden Ansprüche, wobei der Implantattreiber dazu ausgelegt ist, durch die glatt gebohrte Buchse (14) zu verlaufen.

## Revendications

**1.** Ensemble pilote d'implant permettant de placer un implant selon une position d'implant et d'implant numérique à planification virtuelle dans la mâchoire d'un patient, ledit ensemble pilote d'implant comprenant au moins un pilote d'implant (2) et au moins un gabarit chirurgical (12), ledit pilote d'implant comportant un ou plusieurs premiers repères, et étant adapté pour être aligné avec le gabarit chirurgical, le gabarit chirurgical comportant un élément de guidage (14) et comportant un ou plusieurs seconds repères, de sorte que l'alignement d'un ou plusieurs premiers repères sur le pilote d'implant lorsqu'il est relié à l'implant et d'un ou plusieurs seconds repères sur le gabarit chirurgical définisse un déplacement en spirale de l'implant lorsqu'il est inséré dans un os de la mâchoire, le pilote d'implant et le gabarit chirurgical comprenant également au moins un repère parmi lesdits un ou plusieurs premiers et au moins un repère parmi lesdits un ou plusieurs seconds repères de manière à indiquer la position de rotation ou de symétrie de rotation du pilote d'implant telle que définie par la position d'implant numérique à planification virtuelle, grâce à quoi l'élément de guidage est une douille (14) avec un alésage lisse ; lesdits un ou plusieurs premiers repères comprenant un filetage (4) dans ou sur une surface externe du pilote d'implant ; lesdits un ou plusieurs seconds repères comprenant un dispositif de suivi de filetage (10) permettant de suivre le filetage qui se trouve dans ou sur la surface externe du pilote d'implant ; **caractérisé en ce que** le dispositif de suivi de filetage (10) comprend une ou plusieurs broches qui sont amovibles ou rétractables par rapport à l'élément de guidage du gabarit chirurgical.

**2.** Ensemble outil de serrage d'implant selon la revendication 1, au moins l'un desdits un ou plusieurs premiers et au moins l'un desdits un ou plusieurs seconds repères étant destinés à indiquer la position verticale finale du pilote d'implant.

**3.** Ensemble outil de serrage d'implant selon la revendication 1 ou 2, ladite position d'implant et/ou d'implant numérique à planification virtuelle dans la mâchoire d'un patient étant stockée sur un dispositif de stockage de signal.

**4.** Ensemble pilote d'implant selon l'une quelconque des revendications précédentes, l'un desdits un ou plusieurs premiers repères étant un repère réparti.

**5.** Ensemble pilote d'implant selon l'une quelconque des revendications précédentes, ledit filetage présentant un début de filetage (6) et une entrée de filetage (8).

**6.** Pilote d'implant selon l'une quelconque des revendications précédentes, ledit pilote d'implant étant adapté pour passer à travers ladite douille à alésage lisse (14).

# Fig. 1A

# Fig. 2A

Fig. 1B

Fig. 2B

Fig. 3

Fig. 4

Fig. 5

Fig.6

Fig. 7

Fig. 8

**REFERENCES CITED IN THE DESCRIPTION**

**Patent documents cited in the description**

- WO 2010125593 A
- EP 2153797 A
- WO 2007129955 A